# EUROPEAN PATENT APPLICATION

(11) **EP 1 829 927 A1**
(43) Date of publication of application: **05.09.2007**
(21) Application number: 05811615.3
(22) Date of filing: 02.12.2005
(51) Int. Cl.: C08L 69/00, C08L 71/02, A61L 31/00, A61L 29/00

(54) **POLYCARBONATE RESIN COMPOSITION AND MEDICAL INSTRUMENT COMPRISING THE SAME**

(30) Priority: 24.12.2004 JP 2004373994; 21.01.2005 JP 2005013829; 21.01.2005 JP 2005013832
(71) Applicant: MITSUBISHI ENGINEERING-PLASTICS CORPORATION, Tokyo 104-0031 (JP)
(72) Inventor: TAMURA, Masaki, Hiratsuka-shi, Kanagawa 2540016 (JP)
(74) Representative: HOFFMANN EITLE
(86) International application number: PCT/JP2005/022184
(87) International publication number: WO 2006/067946

(57) **Abstract**

The present invention relates to a polycarbonate resin composition which comprises an aromatic polycarbonate resin (A) and a colorant (B) and which has such a property that a molded test specimen comprising said polycarbonate resin composition and having a thickness of 3 mm is exposed to 25 kGy of a cobalt-60 gamma radiation, and the b value of test specimen measured by the Hunter's Lab method after 7 days from the exposure is not more than 2. A molded product comprising the said polycarbonate resin composition is invisible in the yellow discoloration and excellent in the transparency even though it is exposed to 25 kGy of a cobalt-60 gamma radiation for sterilization, especially excellent in such a transparency that the fluid level and color of content such as a medicinal solution or blood in a medical appliance can be easily confirmed, and also is small in mechanical properties deterioration. Therefore, the molded product is suitably used for medical appliances.

## Description

### TECHNICAL FIELD OF THE INVENTION

The present invention relates to a polycarbonate resin composition and medical appliances comprising thereof, and more particularly it relates to a polycarbonate resin composition and medical appliances comprising thereof which are small in the change of color thereof and small in the deterioration of mechanical strength such as impact strength even though they are subjected to an exposure of ionizing radiation, and which are capable of easily confirming the fluid level and color of content such as a medicinal solution or blood in a medical appliance.

### BACKGROUND ART OF THE INVENTION

Polycarbonate resins have been widely utilized in the fields of medical appliances such as an injection syringe, surgical appliances, appliances for an operation and containers for packaging thereof, an artificial lung, artificial dialyzer, an anesthetic inhaler, a vein connector or accessories, a hemo-centrifugal equipment, or the like because the polycarbonate resins have excellent heat resistance, transparency, sanitary properties and mechanical strength. In the medical appliances completely sterilization upon use is generally required. Specifically, the sterilization of these medical appliances has been carried out by an ethyleneoxide gas sterilization process, a high-pressure steam sterilization process in an autoclave, or a sterilization process using an ionizing radiation such as a gamma radiation or an electron beam. Among these sterilization processes, the ethyleneoxide gas sterilization process has posed such problems as toxicity of ethyleneoxide itself, instability, or environmental pollution upon disposal thereof. Similarly, the high-pressure steam sterilization process has disadvantages such as deterioration of polycarbonate resin by high temperature treatment, high energy cost and necessity of a drying step before use because of remaining moisture after the sterilization. Under these circumstances, a recent tendency is such that the sterilization process using an ionizing radiation has been predominately adopted because the sterilization process is relatively inexpensive and can be performed at a low temperature.

However, in the sterilization process using an ionizing radiation, there is such a problem that when exposed to the ionizing radiation for sterilization thereof, the polycarbonate resin suffers from yellow discoloration and the appearance is deteriorated so that it becomes difficult to confirm the fluid level and color of content such as a medicinal solution or blood in a medical appliance. And also, there is such a problem that the medical appliances which have been exposed to the ionizing radiation for sterilization thereof are deteriorated in mechanical strength so that they are easily broken when dropping the medical appliances by mistake or screwing up with a screw.

As means for preventing from the yellow discoloration of polycarbonate by the exposure of ionizing radiation, there have been proposed various methods which are mostly methods of blending an additive (named as a yellow discoloration inhibitor) and methods of modifying the polycarbonate resin by introducing a specific group. As the yellow discoloration inhibitor, there have been proposed polyetherpolyols or alkylethers thereof (refer to Patent Document 1), compounds having benzylocy or benzylthio back born (refer to Patent Document 2), specific triazine-based compounds (refer to Patent Document 3), combination of aromatic hydrcarbon-aldehyde resins and compounds having aryloxy group, arylcarbonyl group, or the like (refer to Patent Document 4), or the like. As the modification method of polycarbonate resin, there have been proposed a specific polycarbonate resin in which hydroxybenzophenone having no halogen atom is introduced to the end group thereof (refer to Patent Document 5), a polycarbonate resin composition comprising polycarbonate substantially having no unsaturated carbon-carbon bond at the end of chain which is capable of cleavage by the exposure of ionizing radiation, and dibenzyl ether or p-(α,α'-dibenzyloxy)xylylene (refer to Patent Document 6), or the like. However, the above yellow discoloration inhibitors and polycarbonate resin composition comprising the modified polycarbonate still have such problems that the yellow discoloration cannot be sufficiently prevented or otherwise, if the amounts of the compounds used therein increases to an extent enough in order to prevent the yellow discoloration, the other properties such as mechanical properties thereof are markedly deteriorated. Therefore, these methods have little practical use. Further, in the above known references, although there are disclosed data showing reduction of yellow discoloration by the exposure of ionizing radiation, there is no concrete description for the mechanical strength. Still further, in the above known references, there is no concrete description for transparency which affects importantly to confirmation of the fluid level and color of content such as a medicinal solution or blood in a medical appliance, therefore there is no recognition of the importance thereof.

Patent Document 1: Japanese Patent Application Laid-Open (KOKAI) No. 62-135556

Patent Document 2: Japanese Patent Application Laid-Open (KOKAI) No. 8-225732

Patent Document 3: Japanese Patent Application Laid-Open (KOKAI) No. 2001-72851

Patent Document 4: Japanese Patent Application Laid-Open (KOKAI) No. 9-25404

Patent Document 5: Japanese Patent Application Laid-Open (KOKAI) No. 8-238309

Patent Document 6: Japanese Patent Application Laid-Open (KOKAI) No. 2002-60616

### DISCLOSURE OF THE INVENTION

### PROBLEM TO BE SOLVED BY THE INVENTION

An object of the present invention is to provided a polycarbonate resin composition having such properties that a molded product thereof is invisible in the yellow discoloration and excellent in transparency even though it is subjected to an exposure of ionizing radiation for sterilization, especially excellent in such a transparency that the fluid level and color of content such as a medicinal solution or blood in a medical appliance can be easily confirmed, and also is small in mechanical properties deterioration, and also medical appliances comprising thereof.

### MEANS FOR SOLVING PROBLEM

As a result of the present inventors' earnest study to solve the above subject, it has been found that a polycarbonate resin composition obtained from blending a specific colorant into an aromatic polycarbonate resin or mixture of an aromatic polycarbonate resin and alkylene glycols and having such a property that when a molded test specimen comprising the polycarbonate resin composition is exposed to the ionizing radiation, the b value of test specimen is not more than 2, is invisible in the yellow discoloration, and further, when the L value of polycarbonate resin composition showing the transparency is not less than 80, it is easy to confirm the fluid level and color of content such as a medicinal solution or blood in a medical appliance, and the deterioration of mechanical strength is small. The present invention has been attained on the basis of the above finding.

In a first aspect of the present invention, there is provided a polycarbonate resin composition which comprises an aromatic polycarbonate resin (A) and a colorant (B) and which has such a property that a molded test specimen comprising said polycarbonate resin composition and having a thickness of 3 mm is exposed to 25 kGy of a cobalt-60 gamma radiation, and the b value of test specimen measured by the Hunter's Lab method after 7 days from the exposure is not more than 2.

In a second aspect of the present invention, there is provided a medical appliance comprising the polycarbonate resin composition as defined in the above aspect.

### EFFECT OF THE INVENTION

The composition according to the present invention using an anti-yellow discoloration agent together with a colorant can attain the effect of preventing yellow discoloration sufficiently in comparison with a composition using only anti-yellow discoloration agent so that the deterioration of other properties by the anti-yellow discoloration agent can be prevented. A molded product produced from the resin composition having the above specific b value according to the present invention is invisible in the yellow discoloration and small in the deterioration of mechanical properties even though it is subjected to an exposure of ionizing radiation. Further, by controlling the L value thereof, the molded product therefrom is excellent in transparency and capable of easy confirmation of the fluid level and color of content such as a medicinal solution or blood in a medical appliance. Therefore, the composition according to the present invention is suitably used for materials of various medical appliances which are subjected to an exposure of ionizing radiation for sterilization.

### PREFERRED EMBODIMENTS FOR CARRYING OUT THE INVENTION

The present invention is explained in detail below. The polycarbonate resin composition according to the present invention comprises an aromatic polycarbonate resin (A) and a colorant (B), and may optionally comprises as the anti-yellow discoloration agent, at least one polyalkylene glycols (C) selected from the group consisting of polyalkylene glycols, ethers of polyalkylene glycol or esters of polyalkylene glycol, compounds having arylalkyl oxy groups or arylalkyl carbonyl groups (D) and aromatic hydrocarbon-aldehyde resins (E), and also may optionally comprises an olefin-based release agent (F) having no unsaturated carbon-carbon bond.

The polycarbonate resin used in the composition according to the present invention may be obtained by reacting an aromatic hydroxy compound or a mixture of the aromatic hydroxy compound and a small amount of ,a polyhydroxy compound with phosgene or diester of carbonic acid and homopolymers or copolymers of a linear or branched thermoplastic aromatic polycarbonate. The method of producing aromatic polycarbonate resin is not limited and known methods such as phosgene method (interfacial polymerization method) and melting method (transesterification method) can be used. The aromatic polycarbonate resin produced by melting method and having end OH groups whose amount is controlled to 50 to 1000 ppm is excellent in wettability to a medicinal solution or blood. Therefore, since medical appliances comprising the above aromatic polycarbonate resin are also excellent in wettability to a medicinal solution or blood and no air bubble generates on the interface between medical appliances and medicinal solution or blood, there is a preferable effect that smooth flow of medicinal solution or blood in the medical appliance can be attained.

As the aromatic dihydroxy compounds which is one of the material for producing a polycarbonate resin, there may be exemplified compounds represented by the following formula (1). 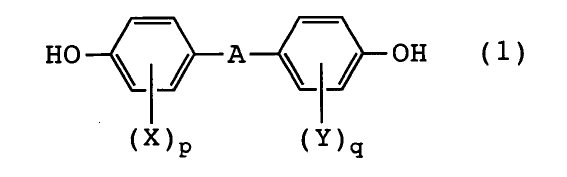 (in the formula, wherein A is a single bond, a divalent straight or branched hydrocarbon group having carbon number of 1 to 10 which may be substituted, or a divalent group represented by -O-, -S-, -CO- or -SO₂-; x and y are independently a hydrocarbon group having carbon number of 1 to 6; and p and q are independently an integer of 0 or 1, proviso that x and y, and p and q may be identical or different each other, respectively.

As typical examples of aromatic dihydroxy compounds represented by the general formula (1), there may be exemplified bis(4-hydroxydiphenyl)methane, 2,2-bis(4-hydroxyphenyl)propane, 2,2-bis(4-hydroxy-3-methylphenyl)propane, 2,2-bis(4-hydroxy-3-t-butylphenyl)propane, 2,2-bis(4-hydroxy-3,5-dimethylphenyl)propane, 4,4-bis(4-hydroxyphenyl)heptane, 1,1-bis(4-hydroxyphenyl)cyclohexane, 4,4'-dihydroxy-biphenyl, 3,3',5,5'-tetramethyl-4,4'-dihydroxy-biphenyl, bis(4-hydroxyphenyl)sulfone, bis(4-hydroxyphenyl) sulfide, bis(4-hydroxyphenyl) ether, bis(4-hydroxyphenyl) ketone, or the like. These aromatic dihydroxy compounds may be used singly or in the form of the mixture thereof. Among them, 2,2-bis(4-hydroxyphenyl)propane (namely, bisphenol A) is preferred.

Branched polycarbonate resins can be obtained by using a polyhydroxy compound (branching agent) such as fluoroglucine and 1,1,1-tri(4-hydroxyphenyl)ethane, which are used as a part of the aromatic dihydroxy compound.

As the carbonic diester which is one of the material for producing the polycarbonate resin (A) produced by the melting method, there may be exemplified compounds represented by the following formula (2). 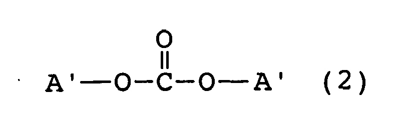 (in the formula (2), wherein A and A' are independently a straight-chain, branched or cyclic monovalent hydrocarbon group having carbon number of 1 to 10 which may be substituted, and A and A' may be the same or different).

As typical examples of the carbonic diester represented by the general formula (2), there are exemplified diphenyl carbonate, substituted diphenyl carbonates such as ditolyl carbonate, and dialkyl carbonate compounds such as dimethyl carbonate, diethyl carbonate and di-t-butyl carbonate. These diphenyl carbonates may be used in combination of any two or more thereof. Among these carbonic diesters, preferred are diphenyl carbonate and substituted diphenyl carbonates.

Further, the above carbonic diesters may be substituted with a dicarboxylic acid or a dicarboxylic acid ester in an amount of preferably not more than 50 mol%, more preferably not more than 30 mol%. As typical examples of the dicarboxylic acid or dicarboxylic acid ester, there are exemplified terephthalic acid, isophthalic acid, diphenyl terephthalate, diphenyl isophthalate, or the like. In case of substituting with such dicarboxylic acid or dicarboxylic acid ester, a polyester carbonate can be obtained.

These carbonic diesters (which include the above substituted dicarboxylic acid or a dicarboxylic acid ester, (the same explanation is given hereinafter)) are usually used in an excess amount to the amount of aromatic dihydroxy compound. Namely, they are used in an amount of 1.001 to 1.3 mol, preferably 1.01 to 1.2 based on the mole of aromatic dihydroxy compound. When the molar ratio of carbonic diesters is less than 1.001 mol, the amount of end OH groups in the polycarbonate resin obtained by melting method increases. Especially, when the amount of end OH groups is more than 1000 ppm, the thermal stability and hydrolysis resistance are deteriorated. When the molar ratio of carbonic diesters is more than 1.3 mol, although the amount of end OH groups reduces, the transesterification rate under the same condition is reduced so that it may be difficult to produce the aromatic polycarbonate resin having the intended molecular weight. Further, in case where the amount of end OH groups is less than 50 ppm, wettability to a medicinal solution or blood may be deteriorated and it is not preferable. Accordingly, in the present invention, it is preferred to use the aromatic polycarbonate resin produced by melting method and having end OH groups whose amount is controlled to 50 to 1000 ppm.

In case where the aromatic polycarbonate resin (A), especially the aromatic polycarbonate resin (A) produced by the melting method, whose end OH groups amount is controlled to 50 to 1000 ppm is produced by the melting method, usually, a catalyst is used. The kind of catalyst is not limited, but usually, basic compounds such as alkaline metal compounds, alkaline earth metal compounds, basic boron compounds, basic phosphor compounds, basic ammonium compounds and amine-based compounds can be used. These compounds may be used in combination of any two or more thereof. The amount of catalyst used is usually 0.05 to 5 µmol, preferably 0.08 to 4 µmol, more preferably 0.1 to 2 µmol. When the amount of catalyst used is smaller than the above range, polymerization activity for producing the aromatic polycarbonate resin having desired molecular weight may not be obtained. When the amount of catalyst used is larger than the above range, the hue of polymer is deteriorated and also chain branching reaction proceeds so that there is a tendency of deteriorating the fluidity at the molding.

The method for producing the aromatic polycarbonate resin (A) by the melting method, especially the aromatic polycarbonate resin (A) produced by the melting method, whose end OH groups amount is controlled to 50 to 1000 ppm is not specifically limited and various known methods can be used. An example of the method is described below. Namely, usually, an aromatic dihydroxy compound and a carbonic diester are mixed with stirring thereof uniformly in a material mixing tank, a catalyst is added thereinto, and polymerization (transesterification) is conducted at the melting condition to produce a polymer. The type of reaction may be any of batch type, continuous type and batch and continuous type. Generally, it is preferred that the reaction is conducted in two or more polymerization tanks, that is, two or more reaction stages, usually multi reaction stages such as 3 to 7 reaction stages.

As the concrete polymerization condition, the reaction temperature is 150 to 320°C, the reaction pressure is ordinary pressure to 2 Pa, the average retention time is 5 to 150 minutes, and the reaction temperature is step-by-step raised to more higher temperature and the reaction pressure is step-by-step reduced to more higher vacuum within the above reaction condition at each reaction tank so that the removing of phenol by-produced with the reaction proceeding is more effective. Incidentally, in order to prevent from deterioration of obtained aromatic polycarbonate qualities such as hue, the reaction condition is preferably set to more lower temperature and more shorter retention time as possible.

The aromatic polycarbonate resin (A) used in the present invention has a viscosity-average molecular weight of preferably 15,000 to 40,000, more preferably 20,000 to 30,000 calculated from the solution viscosity measured at 25°C in terms of a solution viscosity using methylene chloride as a solvent. When the viscosity-average molecular weight is less than 15,000, the mechanical strength thereof may be poor and when the viscosity-average molecular weight is more than 40,000, moldability thereof may be deteriorated.

As the colorant (B) used in the present invention, there are exemplified azo-based colorants, anthraquinone-based colorants, indigo-based colorants, triphenylmethane-based colorants, xanthene-based colorants, or the like. As the concrete examples thereof, there are exemplified D-BLUE-G produced by Mitsubishi Chemical Corporation, M-BLUE-2R and M-Violet-3R produced by Bayer AG, or the like. The amount of colorant (B) blended in the composition according to the present invention is such an amount that a molded test specimen which comprises the composition comprising the aromatic polycarbonate (A), colorant (B) and optional component of anti-yellow discoloration agent explained below in the prescribed amount and which has a thickness of 3 mm is exposed to 25 kGy of a cobalt-60 gamma radiation, after exposure, the test specimen is kept in the atmosphere, at room temperature in a dark room, and the b value of test specimen measured by the Hunter's Lab method after 7 days keeping from the exposure is not more than 2. Therefore, although the amount of colorant (B) blended is different due to the kind and blending amount of anti-yellow discoloration agent as the optional component, and also kind of colorant, the most suitable amount of colorant can be determined by conducting preliminarily experiments.

The rough amount of colorant used is preferably 0.0001 to 0.005 parts by weight (1 to 50 ppm) based on 100 parts by weight of aromatic polycarbonate resin (A), in case of using a blue colorant, it is such amount that the composition has blue color. When the amount of colorant used is less than 0.0001 parts by weight, the b value may be more than 2 and yellow tinge may be visible, or large amount of anti-yellow discoloration agent is required in order to attain the b value of not more than 2 whereby causing the deterioration of mechanical strength.

On the other hand, when the amount of colorant used is too excess amount, there is a possibility that blue tinge may be strong and the L value may be less than 80 whereby deterioration of transparency. The L value of test specimen the composition according to the present invention and having a thickness of 3 mm, which L value is measured by the Hunter's Lab method after 7 days from the exposure of ionizing radiation, is preferably not less than 80. When it has the transparency of L value of not less than 80, in case of using for medical appliances, it is capable of easily confirming the fluid level and color of content such as a medicinal solution or blood in the medical appliance.

Incidentally, some colorants in the above exemplified colorants are so-called bluing agents which are known additives for preventing yellow discoloration. Thus, the amount of colorant added as the bluing agent is about 0.-few ppm which is such amount that the resin containing the bluing agent shows icy color. However, in case of using the colorant in the above amount (0.-few ppm), the effect of preventing yellow discoloration of aromatic polycarbonate by the exposure of ionizing radiation may not be sufficient.

In the resin composition according to the present invention, the aromatic polycarbonate resin (A) and the colorant (B) are essential components. In addition to these essential components, it is preferable to further comprise at least one selected from the group consisting of polyalkylene glycols (C) selected from the group consisting of polyalkylene glycols, ethers of polyalkylene glycol or esters of polyalkylene glycol; compounds having arylalkyl oxy groups or arylalkyl carbonyl groups (D); and aromatic hydrocarbon-aldehyde resins (E), as an agent for preventing yellow discoloration. It is more preferable to use polyalkylene glycols (C), compounds (D) and aromatic hydrocarbon-aldehyde resins (E) in combination. By this addition, the effect of preventing the yellow discoloration upon exposure to an ionizing radiation can be improved and deterioration of transparency and mechanical strength thereof can be prevented.

The polyalkylene glycols (C) used in the composition according to the present invention may be polyalkylene glycols (C-1) or ethers of polyalkylene glycol (C-2) both represented by the following general formula (3), or esters of polyalkylene glycol (C-3) represented by the following general formula (4): 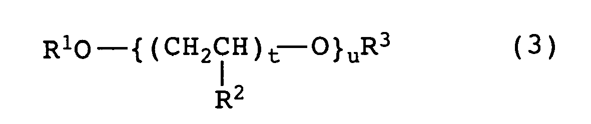 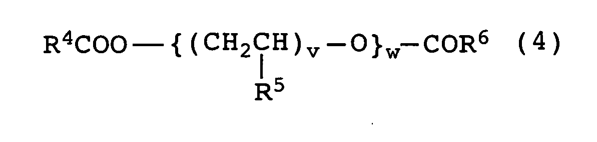 where R¹, R³, R⁴ and R⁶ are independently a hydrogen atom, an alkyl group, cycloalkyl group, alkenyl group, aryl group, arylalkyl group or arylalkenyl group which has each carbon number of 1 to 30, in which the said aromatic ring may be substituted with an alkyl group having carbon number of 1 to 10 or a halogen atom; R² and R⁵ are independently a hydrogen atom or an alkyl group having carbon number of 1 to 4; u is an integer of not less than 1; w is an integer of not less than 2; and t and v are independently an integer of 1 to 10.

In the general formulas (3) and (4), R¹ and R³ are preferably a hydrogen atom, an alkyl group and an arylalkyl group, R³ and R⁶ are preferably an alkyl group and an aryl group, R² and R⁵ are preferably a hydrogen atom and methyl group. u is preferably an integer of 1 to 3000, more preferably 1 to 500, and w is preferably an integer of 1 to 7, more preferably 1 to 5. The polyalkylene glycols (C-1), ethers of polyalkylene glycol (C-2) and esters of polyalkylene glycol (C-3) may be used singly or in the form of the mixture thereof.

As to specific examples of the compounds represented by the formula (3), polyalkylene glycols such as polyethylene glycol, polypropylene glycol and polytetramethylene glycol, ethers of polyalkylene glycol such as polyethylene glycol methylether, polyethylene glycol dimethylether, polyethylene glycol dodecylether, polyethylene glycol benzylether, polyethylene glycol-4-nonylphenylether, polypropylene glycol methylether, polypropylene glycol dimethylether, polypropylene glycol dodecylether, polypropylene glycol benzylether, polypropylene glycol dibenzylether, polypropylene glycol-4-nonylphenylether, or the like may be exemplified.

As to specific examples of the compounds represented by the formula (4), polyethylene glycol acetates, polyethylene glycol-(monoacetate)monopropionate, polyethylene glycol dibutyrate, polyethylene glycol distearate, polyethylene glycol dibenzoate, polyethylene glycol di-2,6-dimethyl-benzoate, polyethylene glycol di-p-tert-butyl-benzoate, polyethylene glycol dicaprylate, polypropylene glycol diacetate, polypropylene glycol-(monoacetate)monopropionate, polypropylene glycol dibutyrate, polypropylene glycol distearate, polypropylene glycol dibenzoate, polypropylene glycol di-2,6-dimethyl-benzoate, polypropylene glycol di-p-tert-butyl-benzoate, polypropylene glycol dicaprylate, or the like may be exemplified.

The blending amount of at least one compound selected from the group polyalkylene glycols (C-1), ethers of polyalkylene glycol (C-2) and esters of polyalkylene glycol (C-3) is usually 0.05 to 5 parts by weight, preferably not more than 3 parts by weight based on 100 parts by weight of the polycarbonate resin. If the blending amount thereof is more than 5 parts by weight, mechanical properties of the resultant polycarbonate resin composition may be unsuitably deteriorated.

The compounds having arylalkyl oxy groups or arylalkyl carbonyl groups (D) used in the composition according to the present invention, are represented by the following formula (5) or (6): 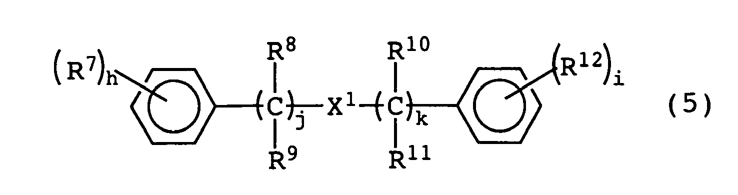 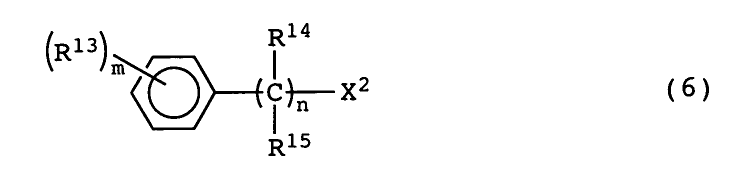 where R⁷, R¹² and R¹³ are independently an alkyl group having carbon number of 1 to 10 or a halogen atom; R⁸, R⁹, R¹⁰, R¹¹, R¹⁴ and R¹⁵ are independently a hydrogen atom, an alkyl group, a cycloalkyl group, an alkylene group, an aryl group, an arylalkyl group, an alkoxy group, an arylalkoxy group or an arylalkoxyalkyl group which has each carbon number of 1 to 30, in which the said aromatic ring may have substituent group(s) of an alkyl group having carbon number of 1 to 10 or a halogen atom; h, i and m are independently an integer of 0 to 5; j, k and n are independently an integer of 1 to 5; X¹ is -O-, -O-(R¹⁶-O)ₓ- or -CO-; X² is -O-(R¹⁷-O)_{y}-R¹⁸, -COR¹⁹, -CO-O-R²⁰ or -O-CO-R²¹; R¹⁶ and R¹⁷ are an alkylene group, an alkenylene group or an arylene group which has each carbon number of 1 to 15, in which the said aromatic ring of arylene group may have substituent group(s) of an alkyl group having carbon number of 1 to 10 or a halogen atom; R¹⁸ is a hydrogen atom, an alkyl group, a cycloalkyl group, an alkylene group or an aryl group which has each carbon number of 1 to 30; R¹⁹ is an alkyl group, a cycloalkyl group, an alkenyl group or an aryl group which has each carbon number of 1 to 30; R²⁰ and R²¹ are an alkyl group, a cycloalkyl group, an alkenyl group, an aryl group or an arylalkyl group which has each carbon number of 1 to 30; and x and y are independently an integer of 1 or more.

In the general formula (6), x and y are independently preferably an integer of 1 to 100. The compound having arylalkyl oxy groups or arylalkyl carbonyl groups may be used singly or in the form of the mixture thereof.

As to specific examples of compound represented by the formula (5), dibenzyl ether, diphenethyl ether, di(1-phenylethyl)ether, dibenzyl ketone, diphenethyl ketone, benzyl(1-phenylethyl) ketone, 1,2-dibenzyloxy ethane, dibenzyloxy polyethylene glycol, dibenzyloxy polytetramethylene glycol, dibenzyloxy polypropylene glycol, or the like may be exemplified. As to specific examples of compound represented by the formula (6), benzylmethyle ketone, benzylphenyl ketone, benzyloxy polyethylene glycol, benzyloxy polytetramethylene glycol, benzyloxy polypropylene glycol, benzyl benzoate, benzyl phenylacetate, or the like like may be exemplified. Incidentally, the above Patent Document 2 discloses that a part of these compounds has the effect of preventing the yellow discoloration of polycarbonate upon exposure to an ionizing radiation.

The blending amount of the compounds having arylalkyl oxy groups or arylalkyl carbonyl groups (D) is usually in the range of 0.01 to 5 parts by weight based on 100 parts by weight of the polycarbonate resin. If the blending amount of the compounds having arylalkyl oxy groups or arylalkyl carbonyl groups (D) is less than 0.01 parts by weight, the aimed effect of preventing the yellow discoloration upon exposure to an ionizing radiation may not be sufficiently achieved. On the other hand, if the blending amount of the compounds having arylalkyl oxy groups or arylalkyl carbonyl groups (D) is more than 5 parts by weight, mechanical properties of the resultant polycarbonate resin composition may be deteriorated. In order to attain both the effect of preventing the yellow discoloration and the deterioration of the mechanical properties under well-balanced conditions, the blending amount of the aromatic compound containing oxy groups or carbonyl groups is more preferably not more than 3 parts by weight based on 100 parts by weight of the polycarbonate resin.

The aromatic hydrocarbon-aldehyde resins (E) used in the composition according to the present invention may be prepared by reacting aromatic hydrocarbon with aldehyde in the presence of an acid catalyst. Specific examples of the aromatic hydrocarbons used for the preparation of the aromatic hydrocarbon-aldehyde resins may include monocyclic aromatic hydrocarbon compounds such as benzene, toluene, ethyl benzene, xylene, methylethyl benzene, trimethyl benzene, tetramethyl benzene, pseudo-cumene or cumene, polycyclic aromatic hydrocarbon compounds such as naphthalene, methyl naphthalene, ethyl naphthalene, dimethyl naphthalene, acenaphthene, anthracene or the like. These aromatic hydrocarbon compounds may be used singly or in the form of the mixture thereof. Among them, the especially preferred aromatic hydrocarbon compounds are toluene, xylene, mesitylene, pseudo-cumene, naphthalene or the like.

Specific examples of the aldehydes used for the preparation of the aromatic hydrocarbon-aldehyde resin (E) may include saturated aliphatic aldehydes such as formaldehyde, acetaldehyde, propionaldehyde, butylaldehyde, iso-butylaldehyde, valeraldehyde, laurinaldehyde or stearinaldehyde; aliphatic polyvalent aldehydes such as glyoxal or succindialdehyde; unsaturated aliphatic aldehydes such as acrolein, crotonaldehyde or propiolaldehyde; aromatic aldehydes such as benzaldehyde, tolylaldehyde, salicylaldehyde, cinnamaldehyde or naphthaldehyde; heterocyclic aldehydes such as furfural; aldehyde derivatives such as methylal, dioxolane, trioxane, tetraoxane, paraformaldehyde, paraldehyde or metaldehyde, or the like.

These aldehydes may be used singly or in the form of the mixture thereof. Among them, the especially preferred aldehydes are formaldehyde, trioxane, paraformaldehyde, acetaldehyde, or the like.

It is preferred that the aromatic hydrocarbon-aldehyde resin (E) contains substantially no acetal group and has such a structure that adjacent aromatic rings are mainly bonded through an alkylene group or alkylene-ether group with each other. The expression "substantially no acetal group" means that the acetal group is contained in an amount of not more than 0.1 mole based on one molecule of the aromatic hydrocarbon-aldehyde resins (E).

Such aromatic hydrocarbon-aldehyde resin (E) containing substantially no acetal group are commercially available. For example, as the commercially available aromatic hydrocarbon-aldehyde resins, NICANOL DS, NICANOL S or NICANOL K (produced by Mitsubishi Gas Chemical Co., Ltd.), may be exemplified. Further, the aromatic hydrocarbon-aldehyde resins can be prepared according to methods disclosed in Japanese Patent Application Laid-open (Kokai) Nos. 60-51133 (1985), 61-23016 (1986), 61-213216 (1986), 63-196616 (1988), 4-224825 (1992), 4-335014 (1992), 5-186544 (1993), 6-136081 (1994) and the like.

It is further preferred that the aromatic hydrocarbon-aldehyde resins (E) used in the present invention, have an oxygen content of not less than 8 % by weight, preferably 9 to 25 % by weight. Such aromatic hydrocarbon-aldehyde resins (E) are commercially available. For example, as the commercially available aromatic hydrocarbon-aldehyde resins (E) having an oxygen content of not less than 8 % by weight, NICANOL H, NICANOL L, NICANOL G or NICANOL Y (produced by Mitsubishi Gas Chemical Co., Ltd.), GENERITE 6010 or GENERITE 5100 (produced by General Petroleum Chemical Co., Ltd.), may be exemplified.

These aromatic hydrocarbon-aldehyde resins (E) may be used singly or in the from of the mixture thereof. The blending amount of the aromatic hydrocarbon-aldehyde resins is in the range of 0.01 to 5 parts by weight, preferably 0.1 to 2 parts by weight based on 100 parts by weight of the polycarbonate resin. If the blending amount of the aromatic hydrocarbon-aldehyde resins (E) is less than 0.01 parts by weight, the aimed effect of preventing the yellow discoloration upon exposure to an ionizing radiation cannot be sufficiently achieved. On the other hand, if the blending amount of the aromatic hydrocarbon-aldehyde resins (E) is more than 5 parts by weight, mechanical properties, heat resistance, etc. of the resultant polycarbonate resin composition are unsuitably deteriorated.

The weight ratio of the aromatic hydrocarbon-aldehyde resins (E) to the aromatic compound containing oxy group or carbonyl group represented by the general formula (I) or (II) is not particularly limited, but is preferably in the range of 10/90 to 90/10, more preferably 20/80 to 80/20.

As described above, when the aromatic compound containing oxy group or carbonyl group and the aromatic hydrocarbon-aldehyde resins (E) are blended at specified blending ratios with the polycarbonate resin, the resultant polycarbonate resin composition can exhibit no deterioration of its mechanical properties and a very low yellow discoloration upon exposure to an ionizing radiation for sterilization thereof

Incidentally, although Patent Document 4 reports that by using the aromatic hydrocarbon-aldehyde resins (E) together with a part of compounds represented by the general formula (5) or (6), there is the effect of preventing yellow discoloration of polycarbonate by the exposure of ionizing radiation. However, in case of using only the combination, it is not necessary attained to sufficiently prevent the yellow discoloration or these compounds are used in a large amount for sufficiently preventing the yellow discoloration so that the mechanical strength thereof may be markedly deteriorated.

The resin composition according to the present invention is capable of preventing the yellow discoloration and deterioration of mechanical strength by the exposure of ionizing radiation and further has transparency. Therefore, it is suitably used for materials of medical appliances exposed to the ionizing radiation for sterilization. When producing the medical appliances from the resin composition according to the present invention, it is preferred to further blend the release agent (F). By blending the release agent, the release resistance at the molding can be reduced, especially, a thin wall molded product can be well produced.

As the release agent (F) used in the present invention, there is no specific limitation as long as known release agents used for aromatic polycarbonate resin, which substantially has no olefin-based unsaturated carbon-carbon double bond. As concrete examples, there are exemplified esters of saturated higher fatty acids such as stearic acid, palmitic acid, myristic acid and behenyl acid, and saturated alcohols such as butyl alcohol, hexyl alcohol, octyl alcohol, nonyl alcohol, lauryl alcohol, myristic alcohol, stearic alcohol, behenyl alcohol, ethylene glycol, propylene glycol, butane diol, hexane diol, glycerin, butane triol, pentane triol, erythritol and pentaerythritol, or partial esters thereof; paraffins ; low molecular polyolefins; other waxes; or the like.

The amount of release agent (F) blended is not more than 3 parts by weight, preferably not more than 1 part by weight based on 100 parts by weight of aromatic polycarbonate resin (A). When the amount of release agent (F) blended is more than 3 parts by weight, there may be problems of deterioration of hydrolysis resistant and mold contamination at the injection molding. The release agent may be used singly or in combination of any two or more thereof.

The polycarbonate resin composition according to the present invention may further contain other additives within adversely affected the effect of the present invention. As the additives, there are exemplified other thermoplastic resins, flame retardants, impact resistance improvers, antistatic agents, phosphorous thermal stabilizers, hindered phenol-based antioxidants, slip agents, anti-blocking agents, anti-fog agents, natural oils, synthetic oils, waxes, organic fillers, inorganic fillers, or the like.

A process for producing the composition according to the present invention is not specifically limited and may comprise blending the colorant (B) and optional components of anti-yellow discoloration agent (polyalkylene glycol (C), compound (D) and aromatic hydrocarbon-aldehyde resin (E)), release agent (F) and other additive(s) into the aromatic polycarbonate resin (A). As the blending method, various known methods by skilled person in the art can be used. Also, there may be used a method of mixing by feeding them into an extrusion hopper quantitatively using a feeder.

The polycarbonate resin composition can be formed into a desired molded product according to a conventional molding method such as an injection-molding method, a blow-molding method, extrusion molding, rotational molding or the like. The molded product comprising the polycarbonate resin composition is small in the color change and mechanical strength such as impact resistance when exposed to an ionizing radiation for sterilization, and when it is used for a medical appliance, it is easy in the fluid level and color of content such as a medicinal solution or blood in a medical appliance. Also, the polycarbonate resin composition further containing release agent is excellent in mold-release properties so that its productivity is enhanced and generation of breakage such as cracking can be prevented. Further, the polycarbonate resin composition according to the present invention using the aromatic polycarbonate resin produced by melting method and having end OH groups whose amount is controlled to 50 to 1000 ppm is excellent in wettability to a medicinal solution or blood. Therefore, since no air bubble generates on the interface between medical appliances and medicinal solution or blood, there is a preferable effect that smooth flow of medicinal solution or blood in the medical appliance can be attained.

Specific examples of the medical appliances to which the polycarbonate resin composition according to the present invention is suitably applied, include an artificial dialyzer, an artificial lung, an anesthetic inhaler, a vein connector or accessories, a hemo-centrifugal bowl, surgical appliances, appliances for an operation room and containers for packaging thereof, tubes for feeding oxygen into blood, connectors for tubes, cardiac probes and injectors, containers for intravenous injection liquid, or the like. The medical appliances according to the present invention are effective for preventing yellow discoloration when exposed to an ionizing radiation such as a gamma radiation, an electron beam or the like for sterilization regardless of kind and irradiation amount, as well as regardless of whether the ionizing radiation for sterilization is conducted under substantially no oxygen atmosphere or oxygen atmosphere.

### EXAMPLES

The present invention is described in more detail below by way of the examples. However, the examples are only illustrative and therefore the present invention is not limited to these examples. The materials and evaluation methods used in each Example and Comparative Example are shown in the following.

### <Materials>

(1) Polycarbonate resin: Iupiron S-3000 produced by Mitsubishi Engineering Plastics Corporation having a viscosity-average molecular weight of 22,000)
(2) Compound having arylalkyloxy group: dibenzyl ether
(4) Compound having arylalkyloxy group: 1,2-dibenzyloxy ethane
(3) Compound having arylalkylcarbonyl group: dibenzyl ketone
(4) Aromatic hydrocarbon-aldehyde resin: NIKANOL Y-50 produced by Mitsubishi Gas Chemical Co., Ltd., oxygen content of 18% by weight
(5) Aromatic hydrocarbon-aldehyde resin: NIKANOL L produced by Mitsubishi Gas Chemical Co., Ltd., oxygen content of 10% by weight
(6) Colorant: M-BLUE-2R produced by Bayer AG
(7) Colorant: M-VIOLET-3R produced by Bayer AG
(8) Polyalkylene glycols compound: polypropylene glycol, molecular weight of 2000, abbreviated as "PPG2000"
(9) Polyalkylene glycols compound: polypropylene glycol distearate, molecular weight of 3000, abbreviated as "PPGST30"
(10) Release agent: pentaerythritol tetrastearate, abbreviated as "PTS"

### <Evaluation methods>

### (1) Color tone

Molded specimens comprising resin compositions described in Examples and Comparative Examples and having thickness of 3 mm were prepared. The obtained molded specimens were exposed to 25 kGy of a cobalt-60 gamma radiation under two conditions: one is kept in the air as it is and the other is kept in a closed system in the presence of deoxidant (trade name: Ageless produced by Mitsubishi Gas Chemical Co., Ltd.) (under deoxidization). After exposure, the L value and b value defined in the Hunter's Lab method before and after the exposure were determined according to JIS K7103 by using a color difference meter (SM-3-CH, manufactured by SUGA Test Instruments Co., Ltd.) to determine the L value and b value defined in the Hunter's Lab method.

### (2) Izod impact resistance

Molded specimens comprising resin compositions described in Examples and Comparative Examples and having thickness of 3 mm and 0.25R notched portion were prepared. The obtained molded specimens were exposed to 25 kGy of a cobalt-60 gamma radiation. After exposure, Izod impact resistances before and after the exposure were measured according to ASTM D256.

### Examples 1 to 10 and Comparative Examples 1 to 4:

An aromatic polycarbonate resin (A), a colorant (B), an anti-yellow discoloration agent (polyalkylene glycol (C), compound (D) and aromatic hydrocarbon-aldehyde resin (E)) and release agent (F) were blended in a tumbler at blending ratios also shown in Table 1. The mixture was fed into a vented single-screw extruder having an screw diameter of 40 mmφ and extruded into pellets at a barrel temperature of 270°C. The thus-prepared pellets were dried in a hot-air drier at a temperature of 120°C for not less than 5 hours. Thereafter, the dried pellets were injection-molded at a resin temperature of 270°C and mold temperature of 80°C to prepare a test specimen for the evaluation of color tone having a diameter of 50 mmφ and a thickness of 3 mm and a test specimen for evaluation of impact resistance having a thickness of 3.3 mm and a 0.25R notched portion.

The thus-obtained test specimens were exposed to 25 kGy of a cobalt-60 gamma radiation under air condition and under deoxidization, respectively, and then the properties the test specimens were evaluated by the above evaluation methods. The results are shown in Tables 1 to 3.

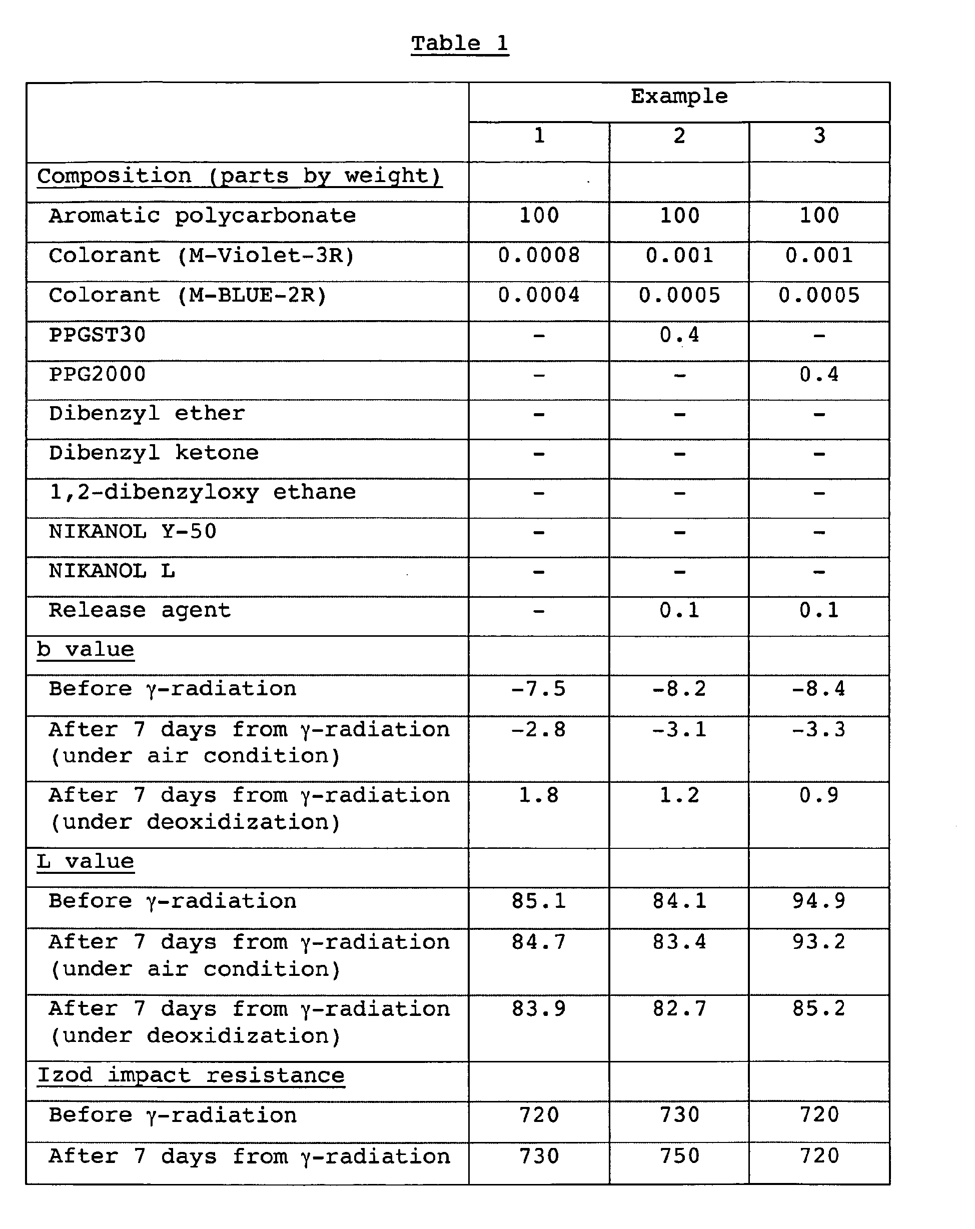

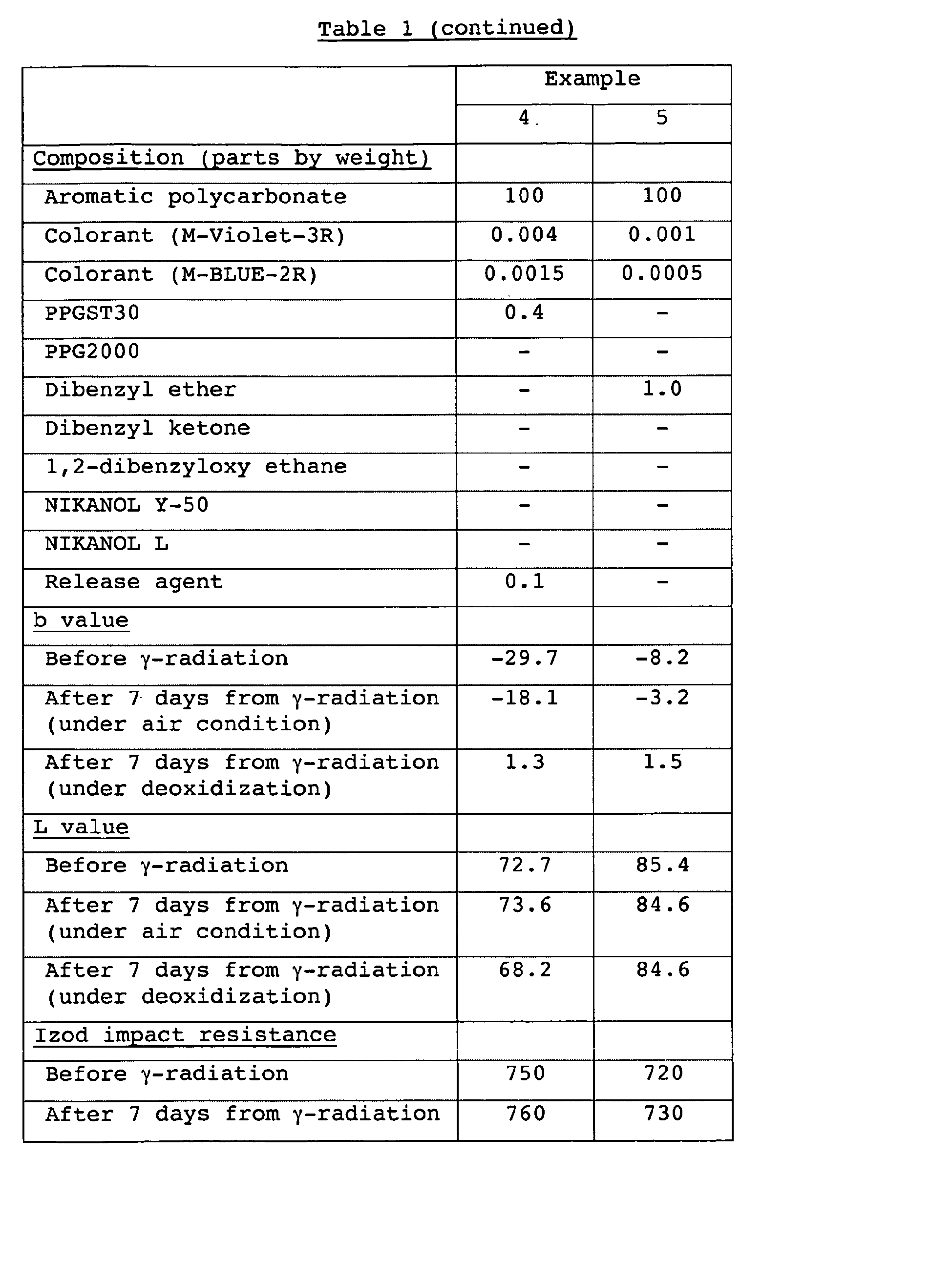

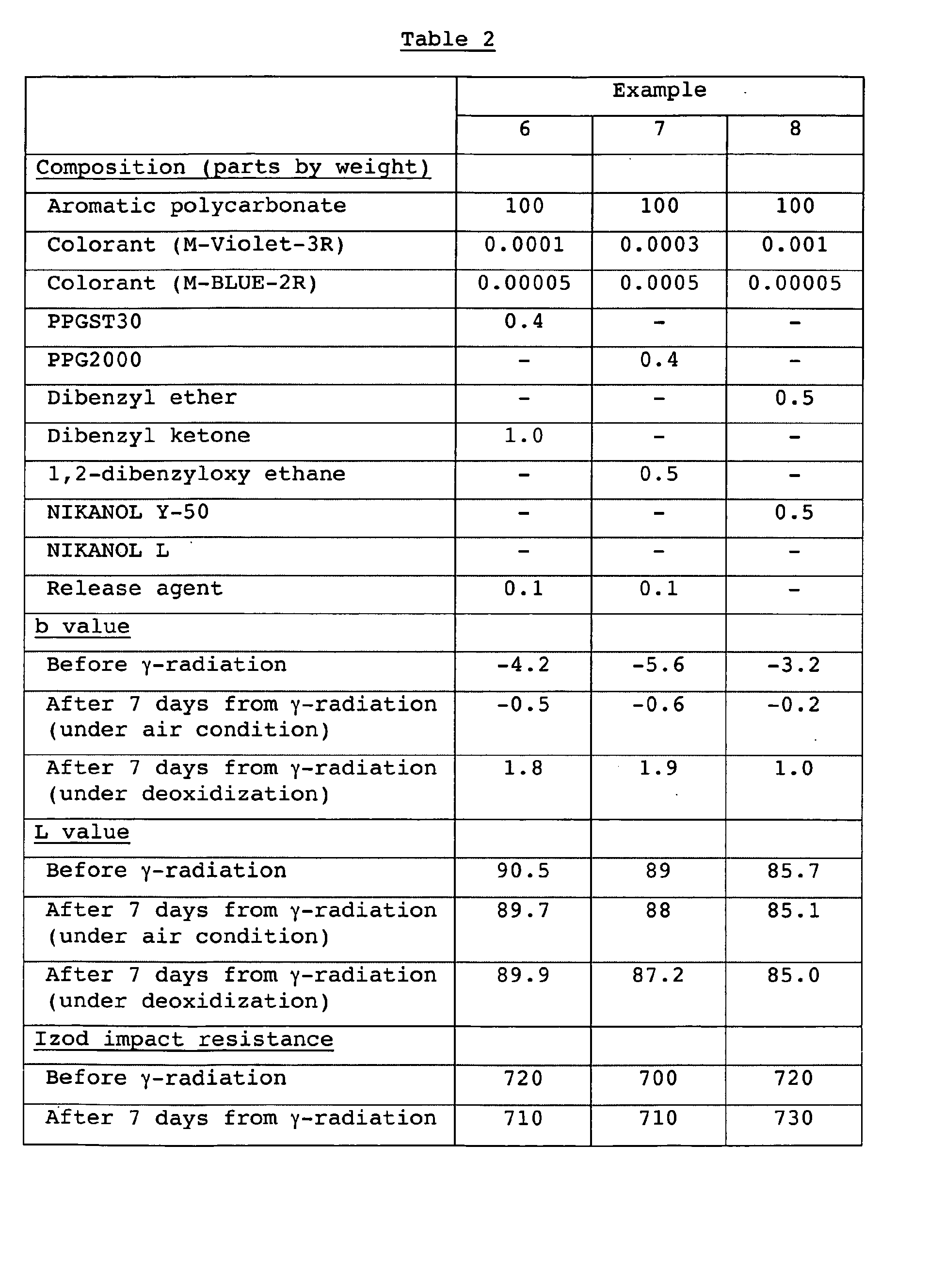

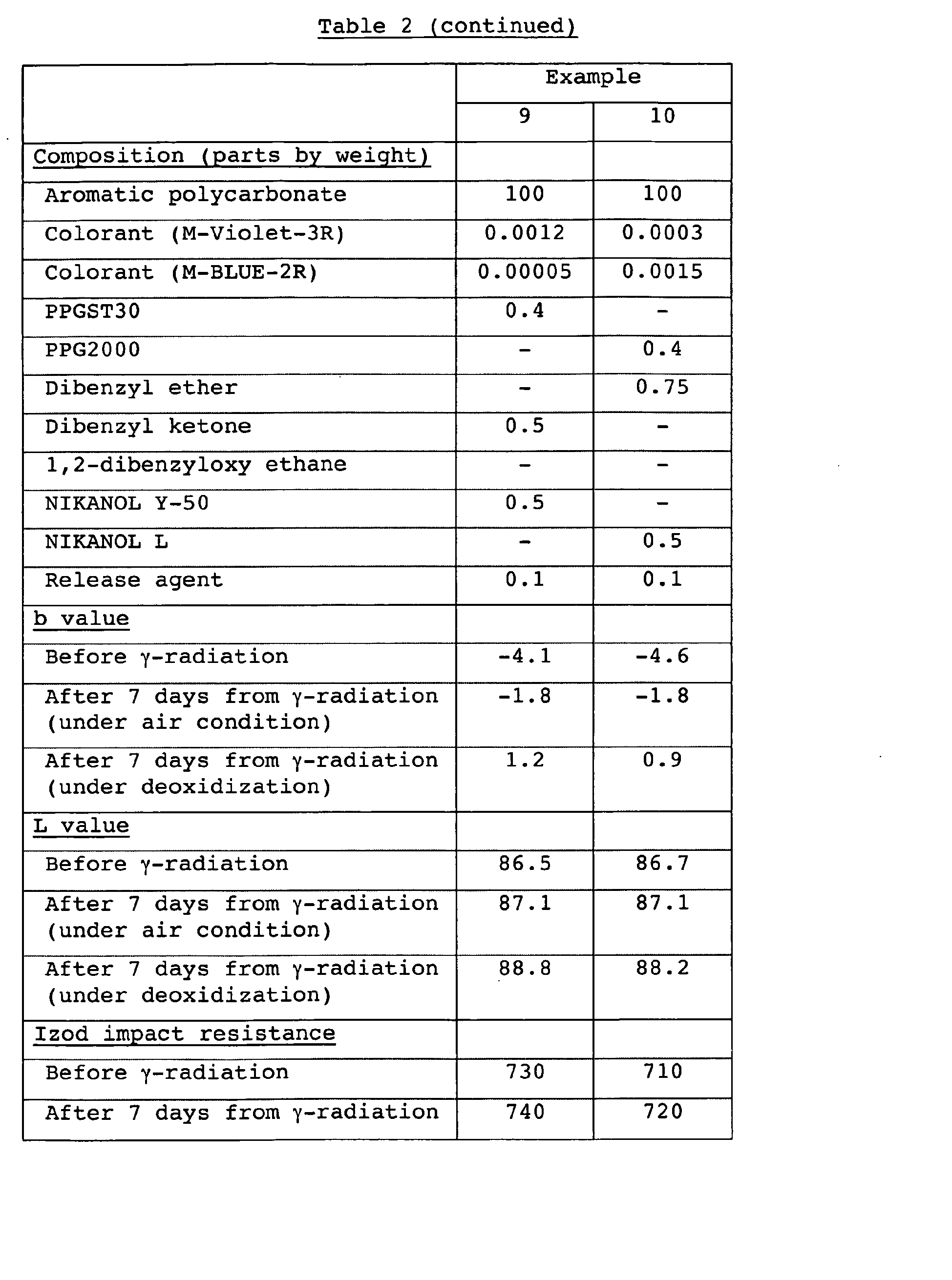

**Table 3**

| | Comparative Example | | | |
|---|---|---|---|---|
| | 1 | 2 | 3 | 4 |
| Composition (parts by weight) | | | | |
| Aromatic polycarbonate | 100 | 100 | 100 | 100 |
| Colorant (M-Violet-3R) | - | 0.00003 | - | - |
| Colorant (M-BLUE-2R) | - | 0.00005 | | |
| PPGST30 | - | 0.4 | 0.4 | 0.4 |
| PPG2000 | 0.4 | - | - | - |
| Dibenzyl ether | - | - | 1.0 | 0.5 |
| Dibenzyl ketone | - | - | - | - |
| 1,2-dibenzyloxy ethane | - | - | - | - |
| NIKANOL Y-50 | - | - | - | 0.5 |
| NIKANOL L | - | - | - | - |
| Release agent | 0.1 | 0.1 | 0.1 | 0.1 |

| b value | | | | |
|---|---|---|---|---|
| Before γ-radiation | 0.5 | -3.8 | 0.6 | 4.5 |
| After 7 days from γ-radiation (under air condition) | 8.2 | -3.2 | 4.6 | 8 |
| After 7 days from γ-radiation (under deoxidization) | 26 | 12 | 7.5 | 10.9 |

| L value | | | | |
|---|---|---|---|---|
| Before γ-radiation | 94.9 | 91.1 | 94.7 | 94.4 |
| After 7 days from γ-radiation (under air condition) | 93.2 | 90.3 | 94.1 | 93.8 |
| After 7 days from γ-radiation (under deoxidization) | 85.2 | 87.7 | 93.8 | 92.6 |

| Izod impact resistance | | | | |
|---|---|---|---|---|
| Before γ-radiation | 730 | 740 | 720 | 720 |
| After 7 days from γ-radiation | 750 | 740 | 710 | 710 |

As seen from Table 1, in the compositions in Examples in which the colorant was blended in the prescribed amount showed that as the color tone, yellow discoloration was not visual after the γ-radiation (b value of not more than 2), they have transparency, the deterioration of Izod impact strength by containing the colorant was small and the mechanical strength was excellent and therefore, they can sufficiently withstand the sterilization treatment by the exposure of ionizing radiation. On the other hand, in the compositions in Comparative Examples in which the colorant was not added or added in the smaller amounts, the b value was more than 2 after the γ-radiation and yellow discoloration was visual.

Although the present invention is described above with respect to embodiments which are considered to be most practical and preferable at the present time, the present invention is not limited to these embodiments, and various changes and modifications will be appropriately made within the scope of claims and a whole of a specification of this application unless departing from the subject matter and concept of the present invention, and it should be construed that the changes and modifications are involved within a technical range of the present invention. The present invention is based on Japanese Patent Application No. 2004-373994 filed on December 24, 2004, Japanese Patent Application No. 2005-13829 filed on January 21, 2005 and Japanese Patent Application No. 2005-13832 filed on January 21, 2005, and the whole content thereof can be incorporated by reference.

### INDUSTRIAL APPLICABLILTY

The polycarbonate resin composition according to the present invention is suitably used for materials of various medical appliances, which are subjected to sterilization treatment by the exposure of ionizing radiation, such as an artificial kidney (artificial dialyzer), an artificial lung, an anesthetic inhaler, a vein connector or accessories, a hemo-centrifugal bowl, surgical appliances, appliances for an operation room and containers for packaging thereof, tubes for feeding oxygen into blood, connectors for tubes, cardiac probes and injectors, containers for intravenous injection liquid, or the like.

## Claims

1. A polycarbonate resin composition which comprises an aromatic polycarbonate resin (A) and a colorant (B) and which has such a property that a molded test specimen comprising said polycarbonate resin composition and having a thickness of 3 mm is exposed to 25 kGy of a cobalt-60 gamma radiation, and the b value of test specimen measured by the Hunter's Lab method after 7 days from the exposure is not more than 2.

2. A polycarbonate resin composition according to claim 1, wherein at least one polyalkylene glycols (C) selected from the group consisting of polyalkylene glycols, ethers of polyalkylene glycol or esters of polyalkylene glycol is contained in an amount of not more than 5 parts by weight based on 100 parts by weight of the aromatic polycarbonate resin (A).

3. A polycarbonate resin composition according to claim 1 or 2, wherein a molded test specimen comprising said polycarbonate resin composition and having a thickness of 3 mm is exposed to 25 kGy of a cobalt-60 gamma radiation, and the L value of test specimen measured by the Hunter's Lab method after 7 days from the exposure is not less than 80.

4. A polycarbonate resin composition according to any one of claims 1 to 3, wherein compounds having arylalkyl oxy groups or arylalkyl carbonyl groups (D) is contained in an amount of 0.01 to 5 parts by weight based on 100 parts by weight of the aromatic polycarbonate resin (A).

5. A polycarbonate resin composition according to claim 4, wherein the compounds having arylalkyl oxy groups or arylalkyl carbonyl groups (D) are at least one compound represented by the following formula (5) or (6): 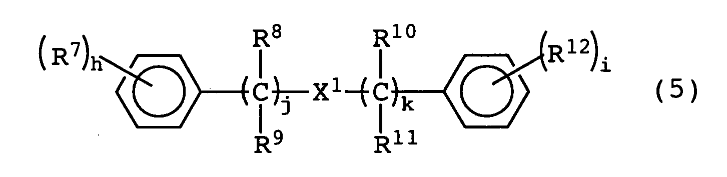 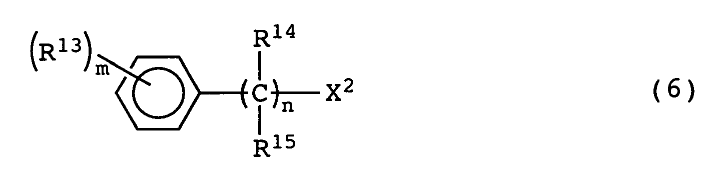 (where R⁷, R¹² and R¹³ are independently an alkyl group having carbon number of 1 to 10 or a halogen atom; R⁸, R⁹, R¹⁰, R¹¹, R¹⁴ and R¹⁵ are independently a hydrogen atom, an alkyl group, a cycloalkyl group, an alkylene group, an aryl group, an arylalkyl group, an alkoxy group, an arylalkoxy group or an arylalkoxyalkyl group which has each carbon number of 1 to 30, in which the said aromatic ring may have substituent group(s) of an alkyl group having carbon number of 1 to 10 or a halogen atom; h, i and m are independently an integer of 0 to 5; j, k and n are independently an integer of 1 to 5; X¹ is -O-, -O-(R¹⁶-O)ₓ- or -CO-; X² is -O-(R¹⁷-O)_{y}-R¹⁸, -COR¹⁹, -CO-O-R²⁰ or -O-CO-R²¹; R¹⁶ and R¹⁷ are an alkylene group, an alkenylene group or an arylene group which has each carbon number of 1 to 15, in which the said aromatic ring of arylene group may have substituent group(s) of an alkyl group having carbon number of 1 to 10 or a halogen atom; R¹⁸ is a hydrogen atom, an alkyl group, a cycloalkyl group, an alkylene group or an aryl group which has each carbon number of 1 to 30; R¹⁹ is an alkyl group, a cycloalkyl group, an alkenyl group or an aryl group which has each carbon number of 1 to 30; R²⁰ and R²¹ are an alkyl group, a cycloalkyl group, an alkenyl group, an aryl group or an arylalkyl group which has each carbon number of 1 to 30; and x and y are independently an integer of 1 or more.

6. A polycarbonate resin composition according to any one of claims 1 to 5, wherein aromatic hydrocarbon-aldehyde resins (E) is contained in an amount of 0.01 to 5 parts by weight based on 100 parts by weight of the aromatic polycarbonate resin (A).

7. A polycarbonate resin composition according to any one of claims 1 to 6, further containing a release agent (F) having no olefin-based unsaturated carbon-carbon bond in an amount of not more than 3 parts by weight based on 100 parts by weight of aromatic polycarbonate resin (A).

8. A medical appliance comprising the polycarbonate resin composition as defined in any one of claims 1 to 7.
